(19) Europäisches Patentamt
European Patent Office
Office européen des brevets

(11) Veröffentlichungsnummer: **0 005 523**
**B1**

# (12) EUROPÄISCHE PATENTSCHRIFT

(45) Veröffentlichungstag der Patentschrift: **26.08.81**

(51) Int. Cl.³: **C 07 J 63/00** // A61K31/235

(21) Anmeldenummer: **79101453.3**

(22) Anmeldetag: **11.05.79**

## (54) D-Homosteroide, Verfahren zu deren Herstellung.

(30) Priorität: **12.05.78 CH 5207/78**
**23.02.79 CH 1841/79**

(43) Veröffentlichungstag der Anmeldung: **28.11.79 Patentblatt 79/24**

(45) Bekanntmachung des Hinweises auf die Patenterteilung: **26.08.81 Patentblatt 81/34**

(84) Benannte Vertragsstaaten: **CH DE FR GB NL**

(56) Entgegenhaltungen:

**EP-A-0 001 737**

**Journal of the American Chemical Society 99 (3) 02-02-1977, Washington DC (USA), Ronald Breslow et al. «Selective halogenation of steroids using attached aryl iodide templates», Seiten 905–15**

(73) Patentinhaber: **F. HOFFMANN-LA ROCHE & CO. Aktiengesellschaft, CH-4002 Basel (CH)**

(72) Erfinder: **Alig, Leo, Dr., Liebrütistrasse 32, CH-4303 Kaiseraugst (CH)**
Erfinder: **Fürst, Andor, Dr., Magnolienpark 14, CH-4052 Basel (CH)**
Erfinder: **Müller, Marcel, Dr., Qüellenweg 10, CH-4402 Frenkendorf (CH)**
Erfinder: **Kerb, Ulrich, Dr., Prinzregentenstrasse 7, D-1000 Berlin 31 (DE)**
Erfinder: **Wiechert, Rudolf, Prof. Dr., Petzowerstrasse 5, D-1000 Berlin 39 (DE)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

EP 0 005 523 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# D-Homosteroide, Verfahren zu deren Herstellung

Die Erfindung betrifft D-Homosteroide der Formel I

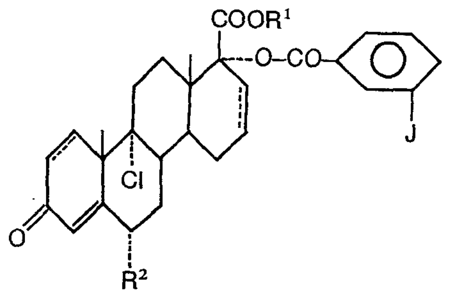

(I)

worin die gestrichelten Bindungen im A- und D-Ring fakultativ sind, $R^1$ Wasserstoff oder niederes Alkyl und $R^2$ Wasserstoff, Fluor oder Methyl bedeuten.

Unter niederem Alkyl sind solche Reste zu verstehen, die sich von niederen Alkanolen mit bis zu 4 Kohlenstoffatomen ableiten. Genannt seien beispielsweise Methyl, Äthyl, Propyl, Isopropyl, Butyl und Isobutyl. Eine bevorzugte Gruppe von D-Homosteroiden der Formel I sind die D-Homosteroide mit gesättigtem D-Ring.

Die erfindungsgemässen Verbindungen sind in erster Linie Zwischenprodukte zur Herstellung von pharmakologisch wertvollen Substanzen. Sie sind aber auch selbst schon pharmakologisch, z. B. hormonal, wirksam.

Beispiele von Verbindungen der Formel I sind
9α-Chlor-17a-(m-jodbenzoyloxy)-3-oxo-D-homo-androst-4-en-17aβ-carbonsäure,
9α-Chlor-17a-(m-jodbenzoyloxy)-6α-methyl-3-oxo-D-homoandrost-4-en-17aβ-carbonsäure,
9α-Chlor-6α-fluor-17a-(m-jodbenzoyloxy)-3-oxo-D-homoandrost-4-en-17aβ-carbonsäure,
9α-Chlor-17a-(m-jodbenzoyloxy)-3-oxo-D-homoandrosta-1,4-dien-17aβ-carbonsäure,
9α-Chlor-17a-(m-jodbenzoyloxy)-6α-methyl-3-oxo-D-homoandrosta-1,4-dien-17aβ-carbonsäure,
9α-Chlor-6α-fluor-17a-(m-jodbenzoyloxy)-3-oxo-D-homoandrosta-1,4-dien-17aβ-carbonsäure,
9α-Chlor-17a-(m-jodbenzoyloxy)-3-oxo-D-homoandrost-4,16-dien-17aβ-carbonsäure,
9α-Chlor-17a-(m-jodbenzoyloxy)-3-oxo-D-homoandrosta-1,4,16-trien-17aβ-carbonsäure,
9α-Chlor-6α-fluor-17a-(m-jodbenzoyloxy)-3-oxo-D-homoandrost-4,16-dien-17aβ-carbonsäure,
9α-Chlor-17a-(m-jodbenzoyloxy)-6α-methyl-3-oxo-D-homoandrosta-1,4,16-trien-17aβ-carbonsäure,
und die Methyl-, Äthyl-, Propyl- und Butylester dieser Verbindungen.

Die erfindungsgemässen Verbindungen der Formel I haben insbesondere den Vorteil, dass sie einen leichten Zugang zu den 11β-Hydroxy-D-homosteroiden bieten, die bisher durch mikrobiologische Hydroxylierung hergestellt wurden. Während bei mikrobiologischen Methoden aufwendige Vorkehrungen getroffen werden müssen (Anzüchten der Mikroorganismen, Sterilität aller Arbeitsmittel, grosse Volumina), können die 11β-Hydroxy-D-homosteroide durch technisch einfach zu realisierende Verfahrensschritte ausgehend von den D-Homosteroiden der Formel I hergestellt werden. So erhält man nach Abspaltung des 9α-Chlors aus den D-Homosteroiden der Formel I nach an sich bekannten Methoden, wie z. B. mit Silberperchlorat in der Wärme, mit einem Alkali- oder Erdalkalimetallcarbonat in Dimethylformamid oder einer organischen Base, wie Collidin, Lutidin und Pyridin, oder mit einem Alkalihydroxyd in einem alkoholischen Lösungsmittel die entsprechenden $\Delta^{9(11)}$-D-Homosteroide. Bei der letzteren Methode werden gleichzeitig auch gegebenenfalls vorhandene Estergruppen gespalten. Die so erhaltenen $\Delta^{9(11)}$-D-Homosteroide sind wertvolle Ausgangsmaterialien für die Herstellung von bekannten 9α,11β-Dihalo-D-homosteroiden oder 11β-Hydroxy-D-homosteroiden, wie z. B. die aus der deutschen Offenlegungsschrift 2 614 079 bekannten D-Homosteroide.

Die 9α,11β-Dihalo-D-homosteroide werden bekanntlich so erhalten, dass man an die $\Delta^{9(11)}$-Verbindung Bromfluor, Chlorfluor oder Chlor addiert. Die 11β-Hydroxy-9α-fluor-D-homosteroide werden bekanntlich so erhalten, dass man an die entsprechende $\Delta^{9(11)}$-Verbindung unterbromige Säure addiert, das erhaltene 11β-Hydroxy-9α-brom-D-homosteroid durch Bromwasserstoffabspaltung in das 9β,11β-Oxide-D-homosteroid überführt und den Epoxidring mit Fluorwasserstoff wieder öffnet. Um zu den 11β-Hydroxy-D-homosteroiden zu gelangen, kann man auch das 11β-Hydroxy-9α-brom-D-homosteroid mit Tributylzinnhydrid, Raney-Nickel oder Chrom-IIchlorid debromieren.

Die Erfindung betrifft des weiteren ein Verfahren zur Herstellung von Verbindungen der Formel I, welches dadurch gekennzeichnet ist, dass man ein D-Homosteroid der Formel II

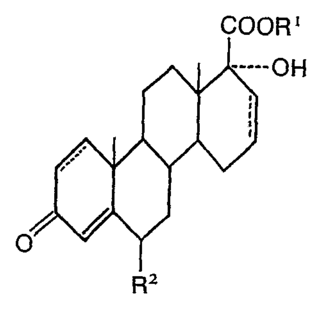

(II)

worin $R^1$, $R^2$ und die gestrichelten Bindungen im A- und D-Ring die gleiche Bedeutung wie in Formel I haben,

a) mit einem m-Jodbenzoylierungsmittel verestert,

b) den so erhaltenen 17aα-m-Jodbenzoylester in Gegenwart von Chlor, von Sulfurylchlorid oder von Jodbenzoldichlorid mit langwelligem UV-Licht bestrahlt oder in Gegenwart eines Radikalbildners erhitzt und gewünschtenfalls eine freie 17a$\beta$-Carboxygruppe vor oder nach der Verfahrensstufe b) verestert.

Aus den Arbeiten von Breslow et al., vgl. J. Amer. Chem. Soc. 96 (1974) 1973, ibid. 96 (1974) 6791 ist bekannt, dass man bei in 3$\alpha$-Stellung veresterten Steroiden mit Jodbenzoldichlorid unter Lichteinfluss das tertiäre Kohlenstoffatom in 9-Stellung chlorieren und anschliessend Chlorwasserstoff unter Ausbildung einer 9,11-Doppelbindung wieder abspalten kann. Dieses Verfahren hat jedoch den Nachteil, dass es allein auf solche Steroide anwendbar ist, die keine Carboxygruppe oder keine ungeschützte Carbonylgruppe in der 17$\beta$-Seitenkette aufweisen. Nach dem erfindungsgemässen Verfahren werden jedoch selektiv 9$\alpha$-Chlor-3-oxo-D-homoandrost-4-en-17a$\beta$-carbonsäuren erhalten. Es war ferner durchaus überraschend, dass die Chlorierung selektiv in 9-Stellung erfolgt, da aus den Arbeiten von Halpern, vgl. Chem. & Ind., 1962, 1571, bekannt war, dass Steroide mit Doppelbindungen mit Jodbenzoldichlorid zu den entsprechenden $\alpha$-Dichlorsteroiden reagieren.

Das erfindungsgemässe Verfahren wird zweckmässigerweise so durchgeführt, dass man die Ausgangsstoffe der Formel II mit einem m-Jodbenzoylierungsmittel, wie m-Jodbenzoylchlorid oder -anhydrid, in Gegenwart eines säurebindenden Mittels, z. B. Pyridin oder Triäthylamin, oder in Gegenwart eines starken Säurekatalysators, z. B. p-Toluolsulfonsäure, umsetzt. Als Lösungsmittel für die m-Jodbenzoylierung kommen nichthydroxylgruppenhaltige organische Lösungsmittel, z. B. chlorierte Kohlenwasserstoffe, wie Methylenchlorid oder Kohlenwasserstoffe, wie Benzol, in Betracht. Dabei entsteht zunächst ein gemischtes Anhydrid der Steroidcarbonsäure und der m-Jodbenzoesäure, das sauer oder basisch, z. B. mit wässriger Salzsäure bzw. wässrigem Diäthylamin, zum gewünschten 17a$\alpha$-Jodbenzoyloxyderivat der Ausgangsverbindung II gespalten wird.

Die Überführung des so erhaltenen 17a$\alpha$-(m-Jodbenzoyl)-D-homosteroidesters in das entsprechende D-Homosteroid der Formel I wird zweckmässigerweise in einem geeigneten Lösungsmittel durchgeführt. Geeignete Lösungsmittel sind solche, die nicht von dem verwendeten Halogenierungsmittel angegriffen werden, wie halogenierte Kohlenwasserstoffe, z. B. Methylenchlorid, Chloroform, Tetrachlorkohlenstoff, Trichloräthylen oder Dichloräthylen, und aromatische Kohlenwasserstoffe, wie Benzol, Chlorbenzol oder Toluol, die gegebenenfalls auch als Mischungen untereinander verwendet werden können. Zweckmässig wird die Reaktion unter Sauerstoffausschluss in einer Schutzgasatmosphäre ausgeführt. Hierzu leitet man ein inertes Gas, wie Stickstoff oder Argon, in die Reaktionslösung. Die Bestrahlung mit langwelligem UV-Licht kann mit einem im Handel erhältlichen Ultraviolettstrahler, z. B. einer Quecksilberhochdrucklampe, erzeugt werden. Als Radikalbildner können organische Peroxide, wie Dibenzoylperoxide, Cu(I)-Acetat oder Azodiisobutyronitril, verwendet werden. Zweckmässigerweise werden 1 bis 25, vorzugsweise 10 Äquivalente des Radikalbildners für 100 Äquivalente des zu chlorierenden D-Homosteroids, in einem Lösungsmittel, z. B. einem der oben genannten halogenierten Kohlenwasserstoffe verwendet.

Die Veresterung einer freien 17a$\beta$-Carboxygruppe kann nach an sich bekannten Methoden, z. B. mit einem Diazoalkan, wie Diazomethan in Äther, oder durch Umsetzung eines Salzes der 17a$\beta$-Carbonsäure, z. B. eines Alkalisalzes, mit einem Alkylhalogenid, wie Methyljodid bewerkstelligt werden.

Die Ausgangsstoffe der Formel II können, soweit sie nicht bekannt oder nachstehend beschrieben sind, in Analogie zu bekannten oder in den Beispielen beschriebenen Methoden dargestellt werden.

Beispiel 1

3,42 g 17a-Hydroxy-3-oxo-D-homoandrost-4-en-17a$\beta$-carbonsäure, Smp. 261 °C, $[\alpha]_D = +59°$ (Methanol, c = 0,1 %), $\varepsilon_{241} = 13\,600$, welche durch Oxydation von 17a,21-Dihydroxy-D-homo-pregn-4-en-3,20-dion mit Perjodsäure in wässerigem Methanol erhalten wurden, werden während 2 Stunden bei 0 °C mit 4,1 ml m-Jodbenzoylchlorid in 98 ml Methylenchlorid in Gegenwart von Triäthylamin umgesetzt. Anschliessend wird das erhaltene gemischte Anhydrid durch basische Hydrolyse mit wässrigem Diäthylamin in 5 g 17a$\alpha$-(m-Jodbenzoyloxy)-3-oxo-D-homoandrost-4-en-17a$\beta$-carbonsäure, Smp. 213–214° C, $[\alpha]_D = -33°$ (Dioxan, c = 0,1 %), $\varepsilon_{220} = 32\,400$ übergeführt.

218 mg 17a-(m-Jodbenzoyloxy)-3-oxo-D-homoandrost-4-en-17a$\beta$-carbonsäure und 104 mg Jodbenzoldichlorid wurden in 22 ml Chloroform während 5 Minuten mit Argon begast und anschliessend während 30 Minuten mit einer Quecksilberhochdrucklampe bestrahlt. Die Chloroformlösung wurde mit Natriumbisulfitlösung und verdünnter Kochsalzlösung gewaschen, getrocknet und eingedampft. Aus Methylenchlorid wurde kristalline 9$\alpha$-Chlor-17a-(m-jodbenzoyloxy)-3-oxo-D-homoandrost-4-en-17a$\beta$-carbonsäure erhalten, welche nach Umkristallisieren aus Aceton-Hexan bei 232–233 °C schmolz; $[\alpha]_D = -58°$ (c = 0,1 % in Dioxan); $\varepsilon_{220} = 33\,800$.

288 mg 17a-(m-Jodbenzoyloxy)-3-oxo-D-homoandrost-4-en-17a$\beta$-carbonsäure, 151 mg Jodbenzoldichlorid und 12,1 mg Dibenzoylperoxid wurden in 50 ml Tetrachlorkohlenstoff $1^1/_4$ Stunden unter Argon am Rückfluss gekocht. Das Reaktionsgemisch wurde mit Chloroform verdünnt, mit Natriumhydrogensulfitlösung und verdünnter Kochsalzlösung gewaschen, getrocknet und eingedampft. Chromatographie des Rückstandes an Kieselgel mit Äther + 1 % Essigsäure gab 9$\alpha$-Chlor-17a-(m-jodbenzoyloxy)-3-oxo-D-homoandrost-4-en-17a$\beta$-carbonsäure, Smp. 232–233 °C (aus Aceton-Hexan). In diesem Verfahren kann Chloroform anstelle von Tetrachlorkohlenstoff verwendet werden.

740 mg 9$\alpha$-Chlor-17$\alpha$-(m-jodbenzoyloxy)-3-

oxo-D-homoandrost-4-en-17aβ-carbonsäure in 50 ml Aceton wurden mit 400 mg Silberperchlorat in 14 ml Wasser versetzt. Das Gemisch wurde 5 Stunden unter Argon am Rückfluss gekocht. Das Reaktionsgemisch wurde mit 1 ml gesättigter Kochsalzlösung versetzt, filtriert und im Vakuum eingeengt. Der Rückstand wurde in Methylenchlorid zweimal mit verdünnter Natriumchloridlösung gewaschen und die Methylenchloridlösung getrocknet und eingedampft. Chromatographie an Kieselgel mit Äther + 1% Essigsäure gab reine 17a-(m-Jodbenzoyloxy)-3-oxo-D-homoandrost-4,9(11)-dien-17aβ-carbonsäure, Smp. 238–239 °C (aus Aceton-Hexan), $[\alpha]_D$ = –38° (Dioxan, c = 0,1%), $\varepsilon_{220}$ = 35 100.

130 mg 17a-(m-Jodbenzoyloxy)-3-oxo-D-homoandrost-4,9(11)-dien-17aβ-carbonsäure und 860 mg Kaliumhydroxydplätzchen wurden in 4,5 ml Äthylenglykol unter Argon 24 Stunden bei 95 °C gerührt. Nach Zugabe von 1 ml Essigsäure wurde das Reaktionsgemisch auf verdünnte Salzsäure gegossen und dreimal mit Methylenchlorid extrahiert. Die organischen Phasen wurden mit Wasser und verdünnter Kochsalzlösung neutral gewaschen, getrocknet und im Vakuum eingedampft. Chromatographie des Rückstandes an Kieselgel gab 17a-Hydroxy-3-oxo-D-homoandrost-4,9(11)-dien-17aβ-carbonsäure, Smp. 255–256 °C (aus Aceton), $[\alpha]_{365}$ = –296° (c = 0,1% in Dioxan), $\varepsilon_{240}$ = 17 000.

140 mg 9α-Chlor-17a-(m-jodbenzoyloxy)-3-oxo-D-homoandrost-4-en-17aβ-carbonsäure und 920 mg Kaliumhydroxydplätzchen wurden in 5 ml Äthylenglykol 20 Stunden unter Argon bei 95 °C gerührt. Nach Zugabe von 1 ml Essigsäure wurde wie oben angegeben 17a-Hydroxy-3-oxo-D-homoandrost-4,9(11)-dien-17aβ-carbonsäure erhalten.

Beispiel 2

1 g 17a-(m-Jodbenzoyloxy)-3-oxo-D-homoandrost-4-en-17aβ-carbonsäure, 1 g Natriumhydrogencarbonat, 1 ml Methyljodid und 7,5 ml Dimethylacetamid wurden 20 Stunden bei 25 °C gerührt. Das Reaktionsgemisch wurde mit Äther und verdünnter Salzsäure zu 17a-(m-Jodbenzoyloxy)-3-oxo-D-homoandrost-4-en-17aβ-carbonsäuremethylester als nicht kristallinen Schaum, $[\alpha]_D$ –32° (Dioxan, c = 0,1%), $\varepsilon_{221}$ = 34 500 aufgearbeitet.

600 mg 17a-(m-Jodbenzoyloxy)-3-oxo-D-homoandrost-4-en-17aβ-carbonsäuremethylester und 350 mg Jodbenzoldichlorid wurden in 20 ml destilliertem Methylenchlorid gelöst. Nach Durchleiten von Argon während 5 Minuten wurde die Lösung während 10 Minuten mit einer Quecksilberhochdrucklampe bestrahlt. Die Reaktionslösung wurde mit wässriger Natriumbisulfitlösung und verdünnter Kochsalzlösung gewaschen, getrocknet und eingedampft. Chromatographie des Rückstandes an Kieselgel mit Äther-Hexan gab 9α-Chlor-17a-(m-jodbenzoyloxy)-3-oxo-D-homoandrost-4-en-17aβ-carbonsäuremethylester, Smp. 224–225 °C (Aceton-Hexan), $\varepsilon_{22}$ = 35 400, $[\alpha]_D$ = –51° (Dioxan, c = 0,1%).

In Analogie zu Beispiel 1 wurde aus dem 9α-Chlor-17a-(m-jodbenzoyloxy)-3-oxo-D-homoandrost-4-en-17aβ-carbonsäuremethylester über den 17a-(m-Jodbenzoyloxy)-3-oxo-D-homoandrost-4,9(11)-dien-17aβ-carbonsäuremethylester, $\varepsilon_{221}$ = 36 000, $[\alpha]_D$ = –28° (Dioxan, c = 0,1%), die 17a-Hydroxy-3-oxo-D-homoandrosta-4,9(11)-dien-17aβ-carbonsäure, Smp. 255–256 °C, hergestellt.

Beispiel 3

Durch Behandlung von 17a-Hydroxy-3-oxo-D-homoandrosta-1,4-dien-17aβ-carbonsäure mit m-Jodbenzoylchlorid in Methylenchlorid in Gegenwart von Triäthylamin und anschliessende basische Hydrolyse des erhaltenen gemischten Anhydrids erhält man 17a-(m-Jodbenzoyloxy)-3-oxo-D-homoandrosta-1,4-dien-17aβ-carbonsäure, Smp. 210–211 °C, $\varepsilon_{220}$ = 32 800, $[\alpha]_D$ = –40° (Dioxan, c = 0,1%).

700 mg 17a-(m-Jodbenzoyloxy)-3-oxo-D-homoandrosta-1,4-dien-17aβ-carbonsäure und 369 mg Jodbenzoldichlorid wurden in 140 ml Chloroform während 5 Minuten mit Argon gespült und anschliessend 25 Minuten lang mit einer Quecksilberhochdrucklampe bestrahlt. Die Chloroformlösung wurde mit Natriumbisulfitlösung und Kochsalzlösung gewaschen, getrocknet und im Vakuum eingedampft. Aus Aceton-Hexan wurde kristalline 9α-Chlor-17a-(m-jodbenzoyloxy)-3-oxo-D-homoandrosta-1,4-dien-17aβ-carbonsäure erhalten, Smp. 252–253 °C, $[\alpha]_D$ = –28° (c = 0,1% in Dioxan) $\varepsilon_{220}$ = 34 600.

In Analogie zu Beispiel 1 wurde aus der 9α-Chlor-17a-(m-jodbenzoyloxy)-3-oxo-D-homoandrosta-1,4-dien-17aβ-carbonsäure über die 17a-(m-Jodbenzoyloxy)-3-oxo-D-homoandrosta-1,4,9(11)-trien-17aβ-carbonsäure, Smp. 228–229 °C, $[\alpha]_D$ = –90° (Dioxan, c = 0,1%), $\varepsilon_{219}$ = 35 000, die 17a-Hydroxy-3-oxo-D-homoandrosta-1,4,9(11)-trien-17aβ-carbonsäure, Smp. 267 bis 268 °C (Aceton-Hexan), $[\alpha]_D$ = –95° (Dioxan, c = 0,1%), $\varepsilon_{239}$ = 15 500, hergestellt.

Das Ausgangsmaterial kann wie folgt hergestellt werden:

21-Acetoxy-17a-Hydroxy-D-homopregn-4-en-3,20-dion wird mit Selendioxid in t-Butanol zu 21-Acetoxy-17a-hydroxy-D-homopregna-1,4-dien-3,20-dion dehydriert, Smp. 220–221 °C, $[\alpha]_D$ = +102° (Dioxan, c = 0,1%), $\varepsilon_{244}$ = 15 700. Daraus erhält man mit Kaliumcarbonat in wässerigem Methanol 17a,21-Dihydroxy-D-homopregna-1,4-dien-3,20-dion, Smp. 236–237 °C, $[\alpha]_D$ = +76° (Dioxan, c = 0,1%), $\varepsilon_{244}$ = 12 400. Abbau mit Perjodsäure in Methanol-Wasser führt zu 17a-Hydroxy-3-oxo-D-homoandrosta-1,4-dien-17aβ-carbonsäure, Smp. 226–227 °C, $[\alpha]_D$ = +24° (Dioxan, c = 0,1%), $\varepsilon_{246}$ = 14 900.

Beispiel 4

In zu Beispiel 1 analoger Weise wird ausgehend von 6α-Fluor-17a-hydroxy-3-oxo-D-homoandrost-4-en-17aβ-carbonsäure, über 6α-Fluor-17aα-(m-jodbenzoyloxy)-3-oxo-D-homoandrost-4-en-17aβ-carbonsäure, Smp. 216–217 °C, $[\alpha]_D$ = –30°

(Dioxan, c = 0,1%), $\varepsilon_{221}$ = 35 770, die 9α-Chlor-6α-fluor-17aα-(m-jodbenzoyloxy)-3-oxo-D-homoandrost-4-en-17aβ-carbonsäure, Smp. 222–224 °C, $[\alpha]_D$ = –54° (Dioxan, c = 0,1%), $\varepsilon_{220}$ = 31 880 erhalten.

Beispiel 5

In zu den Beispielen 1–3 analoger Weise wird ausgehend von 17a-Hydroxy-3-oxo-D-homoandrost-4,16-dien-17aβ-carbonsäure, über 17a-(m-Jodbenzoyloxy)-3-oxo-D-homoandrost-4,16-dien-17aβ-carbonsäuremethylester, Smp. 171–172 °C, $[\alpha]_D$ = 152° (Dioxan, c = 0,1%), $\varepsilon_{221}$ = 35 810, der 9α-Chlor-17a-(m-jodbenzoyloxy)-3-oxo-D-homoandrost-4,16-dien-17aβ-carbonsäuremethylester, Smp. 108 °C, $[\alpha]_D$ = –134° (Dioxan, c = 0,1%), $\varepsilon_{221}$ = 36 750 erhalten.

**Patentansprüche**

1. D-Homosteroide der Formel I

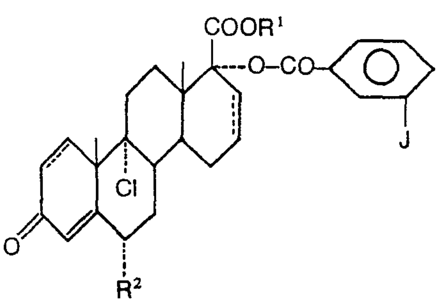

(I)

worin die gestrichelten Bindungen im A- und D-Ring fakultativ sind, $R^1$ Wasserstoff oder niederes Alkyl und $R^2$ Wasserstoff, Fluor oder Methyl bedeuten.

2. D-Homosteroide der Formel I nach Anspruch 1 mit gesättigtem D-Ring.

3. 9α-Chlor-17a-(m-jodbenzoyloxy)-3-oxo-D-homoandrost-4-en-17aβ-carbonsäure,
9α-Chlor-17a-(m-jodbenzoyloxy)-3-oxo-D-homoandrost-4-en-17aβ-carbonsäuremethylester,
9α-Chlor-17a-(m-jodbenzoyloxy)-3-oxo-D-homoandrosta-1,4-dien-17aβ-carbonsäure,
9α-Chlor-6α-fluor-17a-(m-jodbenzoyloxy)-3-oxo-D-homoandrost-4-en-17aβ-carbonsäure,
9α-Chlor-17a-(m-jodbenzoyloxy)-3-oxo-D-homoandrost-4,16-dien-17aβ-carbonsäuremethylester.

4. Verfahren zur Herstellung eines D-Homosteroids der Formel I, dadurch gekennzeichnet, dass man ein D-Homosteroid der Formel II

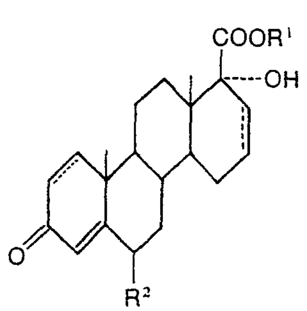

(II)

worin $R^1$, $R^2$ und die gestrichelten Bindungen im A- und D-Ring die gleiche Bedeutung wie in Formel I, Anspruch 1 haben,
a) mit einem m-Jodbenzoylierungsmittel verestert,
b) den so erhaltenen 17aα-m-Jodbenzoylester in Gegenwart von Chlor, von Sulfurylchlorid oder von Jodbenzoldichlorid entweder mit langwelligem UV-Licht bestrahlt oder in Gegenwart eines Radikalbildners erhitzt und gewünschtenfalls eine freie 17aβ-Carboxygruppe vor oder nach Verfahrensstufe b) verestert.

**Claims**

1. D-Homosteroids of the formula

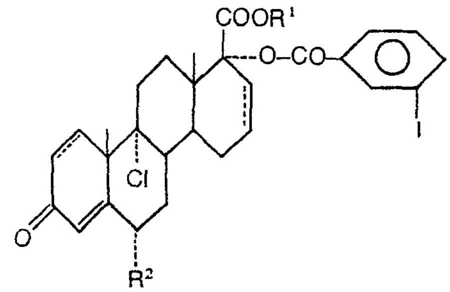

(I)

wherein the dotted bonds in the A- and D-ring are optional, $R^1$ signifies hydrogen or lower alkyl and $R^2$ signifies hydrogen, fluorine or methyl.

2. D-Homosteroids of formula I according to claim 1 with a saturated D-ring.

3. 9α-Chloro-17a-(m-iodobenzoyloxy)-3-oxo-D-homoandrost-4-ene-17aβ-carboxylic acid,
9α-chloro-17a-(m-iodobenzoyloxy)-3-oxo-D-homoandrost-4-ene-17aβ-carboxylic acid methyl ester,
9α-chloro-17a-(m-iodobenzoyloxy)-3-oxo-D-homoandrosta-1,4-diene-17aβ-carboxylic acid,
9α-chloro-6α-fluoro-17a-(m-iodobenzoyloxy)-3-oxo-D-homoandrost-4-ene-17aβ-carboxylic acid,
9α-chloro-17a-(m-iodobenzoyloxy)-3-oxo-D-homoandrost-4,16-diene-17aβ-carboxylic acid methyl ester.

4. Process for the manufacture of a D-homosteroid of formula I, characterised in that a D-homosteroid of formula II

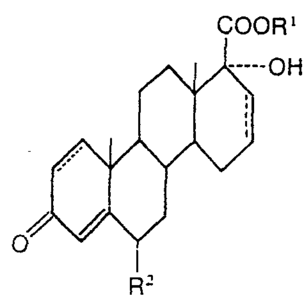

(II)

wherein $R^1$, $R^2$ and the dotted bonds in the A- and D-ring have the same significances as in formula I, claim 1.
a) is treated with a m-iodobenzoylating agent,
b) the thus-obtained 17aα-m-iodobenzoyl ester in the presence of chlorine, of sulphuryl chloride or of iodobenzene dichloride is either irradiated

with long-wave UV-light or heated in the presence of a radical-former and, if desired, a free 17aβ-carboxy group is esterified before or after process step b).

**Revendications**

1. D-homostéroïdes de formule I

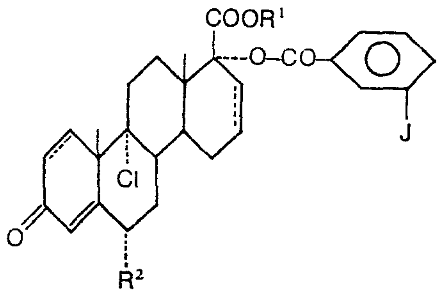

(I)

où les liaisons pointillées dans les noyaux A et D sont facultatives, $R^1$ représente un hydrogène ou un alcoyle inférieur et $R^2$ un hydrogène, un fluor ou un méthyle.

2. D-homostéroïdes de formule I selon la revendication 1 à noyau D saturé.

3. L'acide 9alpha-chloro-17a-(m-iodobenzoyloxy)-3-oxo-D-homoandrost-4-èn-17a bêta-carboxylique,

L'ester méthylique de l'acide 9alpha-chloro-17a-(m-iodobenzoyloxy)-3-oxo-D-homoandrost-4-èn-17a bêta-carboxylique,

L'acide 9alpha-chloro-17a-(m-iodobenzoyloxy)-3-oxo-D-homoandrosta-1,4-dièn-17a bêta-carboxylique,

L'acide 9alpha-chloro-6alpha-fluoro-17a-(m-iodobenzoyloxy)-3-oxo-D-homoandrost-4-èn-17a bêta-carboxylique,

L'ester méthylique de l'acide 9alpha-chloro-17a-(m-iodobenzoyloxy)-3-oxo-D-homoandrost-4,16-dièn-17a bêta-carboxylique.

4. Procédé de préparation d'un D-homostéroïde de formule I, caractérisé en ce que

a) on estérifie avec un agent de m-iodobenzoylation,

b) on irradie avec de la lumière UV à grande longueur d'onde ou on chauffe en présence d'un formateur de radicaux le 17a alpha-m-iodobenzoylester ainsi obtenu en présence de chlore, de chlorure de sulfuryle ou de dichlorure de iodobenzène, et éventuellement on estérifie un groupe 17a bêta-carboxy libre avant ou après l'étape b) du procédé, un D-homostéroïde de formule II

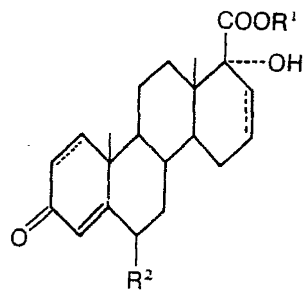

(II)

où $R^1$, $R^2$ et les liaisons pointillées dans les noyaux A et D ont la même signification que dans la formule I.